# EUROPEAN PATENT SPECIFICATION

(11) **EP 2 377 519 B1**
(45) Date of publication and mention of the grant of the patent: **23.03.2016**
(21) Application number: 11173962.9
(22) Date of filing: 18.11.2003
(51) Int. Cl.: A61K 9/00, A61K 9/16, A61K 38/31, A61K 47/10, A61K 47/26, A61K 47/38

(54) **Pharmaceutical Composition Comprising Octreotide Microparticles**
Arzneimittel enthaltend Octreotidmikropartikel
Composition pharmaceutique comprenant des microparticules d'octreotide

(30) Priority: 19.11.2002 GB 0226993; 29.11.2002 GB 0227883
(43) Date of publication of application: 19.10.2011
(62) Divisional of application: 03789053.0
(73) Proprietor: Novartis AG, 4056 Basel (CH); Novartis Pharma GmbH, 1230 Wien (AT)
(72) Inventor: Lambert, Oliver, 68720 Spechbach-le-Haut (FR); Ausborn, Michael, 79540 Lörrach (DE); Petersen, Holger, 79591 Eimeldingen (DE); Loeffler, Rolf, 79110 Freiburg (DE); Bonny, Jean-Daniel, 4414 Füllinsdorf (CH)
(74) Representative: Pfister-Fu, Yixin

(56) References cited:
- EP-A- 0 469 520
- WO-A2-02/49620
- US-A- 5 538 739

## Description

The present invention relates to pharmaceutical compositions, in particular to depot microparticles.

Octreotide acetate microparticles for injectable suspension are commercialized as pharmaceutical compositions under the brand name SANDOSTATIN LAR. These pharmaceutical compositions are indicated for, inter alia, long-term maintenance therapy in acromegalic patients, and treatment of severe diarrhea and flushing associated with malignant carcinoid tumors and vasoactive intestinal peptide tumors (vipoma tumors). The pharmaceutical compositions are normally administered once-a-month. The octreotide is presented as a sterile pharmaceutical composition in a vial which when mixed with a vehicle for suspension such as sterile water becomes a suspension that is administered by an intragluteal injection.

The octreotide acetate microparticles are produced from the acetate salt of octreotide which is distributed throughout a biodegradable poly (DL-lactide-co-glycolide)-glucose star polymer (disclosed in e.g. US patent 5,922,682, the contents of which are incorporated herein by reference). The octreotide acetate microparticles are produced according to the teaching of US patent 5,538,739 (the contents of which are incorporated herein by reference) involving use of silicone oil and heptane. Traces of these starting materials may be detected in the final product.

Until now, no octreotide composition based on linear poly (lactide-co-glycolide) in sustained release form for parenteral administration has reached the market. The present invention provides a sustained release formulation of octreotide which is less expensive than the one available on the market and which is more easy to manufacture.

The present invention provides processes for manufacturing commercially acceptable octreotide acetate microparticles produced from linear poly (lactide-co-glycolide), hereinafter referred to as PLG, which have similar pharmacokinetic characteristics to SANDOSTATIN LAR with acceptable drug loading whilst also of high purity and which may be free from silicone oil and heptane.

In one process for the production of octreotide acetate microparticles by an emulsion process, as defined by claim 1 and also referred to as process C, the improvement comprises the steps of:
a) mixing octreotide acetate in methanol with methylene chloride containing a dissolved linear poly (lactide-co-glycolide) to form a solution; and
b) emulsifying said solution with the extraction medium, e.g. water or preferably an aqueous buffered solution with a stabilizer, e.g. PVA;
c) immediately after the formation of said emulsion, adding all at once said emulsion to an effective amount of an extraction medium, e.g. an aqueous phase, e.g. an aqueous solution of a stabilizing agent, e.g. polyvinylalcohol, to extract said solvent from said microdroplets to form said microparticles;
   and
d) collecting and drying the microparticles, e.g. freeze-drying or drying under vacuum.

According to this process C, octreotide acetate is preferably dissolved in a polar organic solvent miscible with methylene chloride, preferably methanol. The process may be effected as described above.

The process medium of process C can be, e.g. an aqueous phase, e.g. an aqueous solution of PVA, e.g. an aqueous solution of PVA and optionally at least one buffering salt. The process medium can be saturated with the same organic solvent as used to dissolve the polymer in order to stabilize the formation of the emulsion droplets and avoid a too fast extraction of the polymer solvent from the emulsion droplets.

In step a) of process C, the PLG may be dissolved in any of the solvents suitable for PLG dissolution, e.g. ethylacetate, tetrahydrofurane, acetonitrile, methylene chloride, hexafluoroisopropanol, chloroform, acetone. For example, PLG may be dissolved in methylene chloride at a concentration of from about 1% to about 40%, typically about 2-2.5% w/v.

In the processes according to the invention, when the suitable solvent used to dissolve PLG is miscible with water, the suitable polymer solvent may be removed by extraction from the microparticles with water or with an aqueous solution as described above. When the suitable solvent used to dissolve PLG is non- or poorly miscible with water then the polymer solvent may be eliminated by evaporation. In this process according to the invention, defined by claim 2, step a) would be identical as described in the process C of the invention. But for step b), the dispersion or the solution or the emulsion prepared in step a) is mixed with high shear stress with a suitable quantity of to 50 volumes of continuous medium. Then step c) would consist in hardening the microparticles by solvent evaporation under stirring, e.g. 200 rpm at room temperature with constant evacuation of solvent vapors, for 1 up to 10 hours , e.g. 5 hours with optionnaly a heating phase up to 40°C, preferably up to 52°C;
and
step d) would consist in washing the microparticles, e.g. in water, collecting and drying the microparticles, e.g. freeze-drying or drying under vacuum.

The processes may be effected in conventional manner, e.g. high speed stirrers may be used to produce emulsions.

The concentration of octreotide acetate in polar organic solvent or in aqueous solution is preferably from about 1% to about 20% w/v, preferably about 4 to 10% w/v, more preferably about 4 to 7% w/v, even more preferably about 5% w/v. It is preferred to have a homogeneous solution after mixing of the octreotide with the dissolved linear poly (lactide-co-glycolide). Mixture of up to about 20% v/v of (i) a solution containing methanol with (ii) methylene chloride containing a dissolved linear poly (lactide-co-glycolide), e.g. with the concentration of poly(lactide-co-glycolide) in the organic solvent being not more than 20% w/v), may still result in a homogeneous solution, e.g. up to about 20% v/v of solution (i) in a mixture of (i) + (ii). The weight ratio of component (i) to component (ii) is typically about 1:8.

In step b) an emulsion may be produced by dispersing the octreotide acetate/PLG - methylene chloride mixture into an aqueous process medium. The continuous phase which is preferably saturated with the polymer solvent, e.g. methylene chloride.

Prior to the addition of the mixture containing the PLG/octreotide to the process medium, the process medium is preferably saturated with the suitable solvent of the polymer, e.g. methylene chloride to reduce extraction of solvent from the microdroplets during formation of the emulsion. The process medium is then mechanically agitated with devices such as homogenizers, propellers or the like, as the PLG/octreotide mixture is added to the process medium. During this step of the process, no solvent may be generally evaporated or removed from the microdroplets. The temperature at which the emulsion is formed is not particularly critical, except that it may be within a range that will prevent the methylene chloride from boiling or the process medium from gelling or freezing or the octreotide or PLG from degrading. The time required to form an emulsion is quite short. Generally, emulsions may be formed within 30 seconds to 5 minutes, depending upon the stabilizing agent, if any, used and the method of agitation of the process medium. Preferably, a stabilizing agent is present.

Preferably, a stabilizing agent for emulsions produced in microparticle processes is present to prevent agglomeration. The concentration present may affect the final size of the microparticles. Generally, the concentration of the emulsion stabilizing excipient in the process medium will be from 0.01% to about 20% w/v depending on the surfactant, the polymer solvent, and the process medium used. The amount of stabilizing agent is preferably from about 0.025 to about 1 %w/v.

Suitable stabilizing agents include:
A) polyvinyl pyrrolidone: Suitably the molecular weight may vary between 2000 and 20000 daltons. Suitable examples include those commonly known as Povidone K12 F (average molecular weight about 2500 daltons), Povidone K15 (average molecular weight about 8000 daltons) or Povidone K17 (average molecular weight about 10000 daltons). Preferably, the polyvinyl pyrolidone is present in an amount of from about 0.1 to about 20% w/v, e.g. about 5% w/v.
B) carboxymethyl cellulose sodium (NaCMC): Preferably it has a low molecular weight. The viscosity may be, e.g. up to 20 cP or mPa s for a 2% aqueous solution or a viscosity of from 8 to 25 mPa s. Conveniently the degree of substitution is from about0.5 to about 1.45, e.g. 1.15 to about 1.45, e.g. 0.7. Typically the sodium content is about 5 to about 12%, e.g. 10.5% to about 12%.
C) polyvinyl alcohol, herein after referred as PVA: In one embodiment, the polyvinyl alcohol has a molecular weight from about 10000 to about 150000, e.g. from about 10000 to about 90000 daltons, e.g. about 30000 daltons.
   Conveniently, the polyvinyl alcohol has a low viscosity having a dynamic viscosity of from about 3 to about 9 mPa s when measured as a 4% aqueous solution at 20°C or by DIN 53015. Suitably, the polyvinyl alcohol may be obtained from hydrolyzing polyvinyl acetate. Preferably, the content of the polyvinyl acetate is from about 10 to about 90% of the polyvinyl alcohol. Conveniently the degree of hydrolysis is about 85 to about 89%. Typically the residual acetyl content is about 10 to 12 %. Preferred brands include Mowiol 4-88, Mowiol 8-88 and Mowiol 18-88 available from Clariant AG Switzerland.
   Preferably the polyvinyl alcohol is present in an amount of from about 0.1 to about 5%, e.g. 0.5% w/v.
D) gelatin: Preferably, the gelatin is porcine or fish gelatin. Conveniently, the gelatin has a viscosity of about 25 to about 35 cps for a 10% solution at 20°C.
   Typically, pH of a 10% solution is from about 6 to about 7. A suitable brand has a high molecular weight, e.g. Norland high molecular weight fish gelatin obtainable from Norland Products Inc, Cranbury New Jersey USA. Preferably, the gelatin is present in an amount of from about 0.01 to about 5%, e.g. 0.5 % or 0.05%.

Conveniently, polyvinyl alcohol may be used.

In step c) transferring all of the emulsion immediately to a large volume of process medium or other suitable extraction medium to immediately extract the solvent from the microdroplets in the emulsion forms microparticles of the invention.

As soon as an emulsion forms, all of the process medium containing the organic microdroplets is transferred, as quickly as possible, to an extraction medium so that greater than 20% to 30% of the solvent may be immediately removed from the microdroplets, e.g. within 3 minutes. Normally, water is used as the extraction medium but other solvents or oils can also be used. In addition, salts may be added to the extraction medium to adjust its ionic strength or pH. The amount of extraction medium used may be somewhat critical in that sufficient medium must be present to allow approximately immediate extraction of the solvent out of the microdroplets. Accordingly, the volume of the extraction medium will depend on the solvent used to dissolve the wall material and its solubility in the extraction medium. Generally, the volume of the extraction medium should be at least the volume needed to dissolve all of the solvent out of the microdroplets, preferably a volume 10-fold or higher.
In one embodiment, the added water is at a pH of about 7 or higher. Such pH may be adjusted to increase the encapsutation efficiency of the octreotide in the microparticles of the invention.
Preferably, an aqueous sodium dihydrogen phosphate/disodium hydrogen phosphate buffer solution is present
After extraction of all or almost all of the solvent from the microdroplets, e.g. generally within 15 to 30 minutes, the hardened microparticles may be collected by centrifugation, filtration, or the like.

In one embodiment, gelatin is not used and is absent in the microparticles of the invention.

The lactide may be D, L or mixtures thereof, e.g. racemic DL lactide.
Homopolymers, e.g. poly(DL-lactide) homopolymers, may be used. The molecular weight of the homopolymers is from about 7,000 to 25,000 daltons, e.g. 18 000 daltons.

Preferably the polymer used is poly (DL-lactide-glycolide), herein after referred as PLG.
The ratio of lactide to glycolide units in the PLG may vary between wide limits. It is however preferred to have a molar ratio of from 90 to 10 to 40:60 lactide to glycolide units, e.g. (i) 50:50 poly(lactide-glycolide) or (ii) 75:25 poly(lactide-glycolide) or (iii) 65:35 poly(lactide-glycolide).
The polymers may be pure poly(lactide-glycolide) polymers or copolymers with other units. Preferably they are pure poly(lactide-glycolide) polymers.
Typically, the average molecular weight of the PLG is from about 5,000 to about 70,000 daltons, e.g. 13 000, preferably it is from about 30,000 to about 70,000 daltons, especially from about 40,000 to about 60,000 daltons, more especially about 50,000 daltons.
The inherent viscosity of the PLG may vary between wide limits. It is however preferred to be in the range from about 0.1 to about 0.8 dL/g, e.g. from about 0.2 to about 0.8 dL/g in hexafluoroisopropanol or preferably chloroform when measured under standard conditions, e.g. 20°C. A preferred example has a viscosity of from 0.45-0.55 dL/g in chloroform. Preferably, the polymer is amorphous. The linear polymer of the invention is not a star polymer and contains less than 5%, or preferably is free from, star polymers, e.g. a reaction product of a polyol containing at least 3 hydroxyl groups and having a molecular weight of up to 20,000 or a reactive derivative thereof and lactic acid or a reactive derivative thereof and glycolic acid or a functional derivative thereof. These star polymer products are disclosed, e.g.
The linear polymers of the invention may be produced in conventional manner, e.g. using conventional techniques such as polycondensation and ring-opening of dimers. The production may be, e_{.}g_{.} according to the teachings of US patent 3 773,919. The polymer may be a reaction product of lactic acid or a reactive derivative thereof, e.g. D,L-lactide, and glycolic acid or a functional derivative thereof, e.g. glycolide. There may be present a suitable catalyst for the production of linear polymers for example zinc oxide, zinc carbonate, basic zinc carbonate, diethyl zinc, organotin compounds, for example stannous octoate (stannous 2-ethylhexanoate), tributylaluminium, titanium, magnesium or barium compounds or litharge. Stannous octoate (stannous 2-ethylhexanoate) is preferred.

The polymer is preferably obtained from Birmingham Polymers Inc., Birmingham, Alabama, USA.
The octreotide acetate may be produced in conventional manner, e.g. as disclosed in US patent 4,395,403.
Insofar as any aspect of production of the microparticles of the invention are not disclosed herein, such production aspect may be effected in conventional manner or in a manner analogous to known methods.
The amount of octreotide on or near the surface and hence the initial drug burst may be reduced by briefly washing the microparticles of the invention with water, e.g. including a 1/15 molar acetate buffer at pH 4.0 during 5 minutes.
The microparticles of the invention may be dried, e.g. to remove water and other volatiles like methylene chloride.
In the drying step, the microparticles of the invention may be subjected, e.g. to:
1) a freeze-drying process or
2) vacuum of 10⁻² to 50 millibars, e.g. 30 millibars or 0.1 mbar
3) addition of mannitol in powder to the filtered microparticles in a tumbler, under vacuum as in the drying process 2) and heating of 45°C to 55°C, preferably 48 to 54°C, most preferably 50 to 52°C.
The volatile solvent, e.g. methylene chloride, may alternatively be removed from the microparticles in suspension in aqueous suspension preferably a buffered water solution, e.g. potassium/sodium phosphate, optionally under vacuum conditions of the drying process 2. Preferably, the microparticles of the invention may be purged with nitrogen or another inert gas. If desired the microparticles of the invention may be heated, e.g. from 25 to 55°C, preferably from 48 to 54°C. Duration of the drying period may be, e.g. from 2 hours to 5 days.
Hence, the present invention provides a process for the production of octreotide acetate microparticles by an emulsion process, which comprises the step of removing volatile solvents, e.g. methylene chloride.
The resultant microparticles may be free-flowing powders of spherical particles.
The microparticles of the invention contain preferably less than 1% silicone oil, e.g. less than 0.5 or 0.1 %, preferably less than 0.05%, especially less than 0.01%, silicone oil.
The microparticles of the invention include for example less than 0.5%, e.g. less than 0.2%, preferably less than 0.1%, methylene chloride.
The microparticles of the invention include for example less than 0.05%, e.g. less than 0.03%, preferably less than 0.01% especially less than 0.005% or 0.001%, methanol.
The microparticles of the invention include for example less than 3%, e.g. less than 1%, less than 0.1% preferably less than 0.05 or 0.01%, especially less than 0.005%, polyvinyl alcohol. The microparticles of the invention include for example less than 2%, e.g. less than 1 or 2.%, preferably less than 0.1 % heptane, especially less than 0.01% or 0.005% heptane.

The microparticles of this invention may have, e.g. a size range from about 1 to 250, preferably 10 to 200, especially 10 to 130 microns in diameter. Mean diameters may be, e.g. from 30 to 100 microns, e.g. from 30 to 90 microns, e.g. 80 to 100 microns.
The size distribution of the microparticles of the invention has preferably at least one of the following average diameter characteristics:
99% or more smaller than 130 microns
90% or more smaller than 90 microns
80% or more smaller than 70 microns
95% or more greater than 10 microns
based on the average size distribution as measured by conventional light scattering methods.
It is preferred to have a broad size distribution of the microparticles of the invention.

The microparticles of the invention may exhibit a smooth to rough surface.
It is preferred to have a smooth surface in the microparticles of the invention. The smoothness may be determined in conventional manner, e.g. by visual determination by electron microscopy.
The microparticles of the current invention are usually made up of particles of a spherical shape, although microparticles may be irregularly shaped.
Preferably the surface area varies by about 5% from the corresponding surface area of a sphere.
Additionally the content uniformity of a unit dose is excellent. Unit doses may be produced which vary from about 85 to about 115%, e.g. from about 90 to about 110%, or from about 95 to about 105%, of the theoretical dose.
Preferably the microparticles of the invention are dense rather then porous. The porosity may be determined in conventional manner, e.g. by visual determination by BET-nitrogen-sorption / Hg-porosimetry or electron microscopy, e.g. by observing the diameter and extent of channels in a cut microparticle.
Preferably the microparticles of the invention contain less than 4 %, especially less than 3% and preferably less than 2% total octreotide degradation products.

The present invention also provides a pharmaceutical composition comprising microparticles of the invention.

The pharmaceutical composition may be in the dry state. Preferably, the pharmaceutical composition contains a vehicle to facilitate reconstitution. In one embodiment the the vehicle preferably comprises from about 1% to 40% of the pharmaceutical composition. In another embodiment of the invention the microparticles of the invention may comprise from about at least 90 % of the pharmaceutical composition.

A vehicle to facilitate reconstitution may be provided together with the pharmaceutical composition. The vehicle for reconstitution may be provided, e.g. in a separate vial or ampoule or in a separate chamber of a two chamber syringe, e.g. a two chamber syringe consisting of one compartment containing the microparticles and optionally an isotonizing agent, and optionally a surfactant and optionally a viscosity increasing agent, e.g. coated on the microparticles or present as a layer in the compartment containing the microparticles and one compartment containing the vehicle or an aqueous phase, e.g. water for injection, a buffered solution, e.g. a low molarity phosphate bufferred solution at physiologic pH, if all the excipients of the vehicle are already in the chamber together with the microparticles, for suspension of the microparticles.

The microparticles of the invention are hydrophobic. When using water as a vehicle, the microparticles of the invention will not resuspend and they will float on the top of the aqueous phase. Therefore the problem is to find a vehicle to suspend the microparticles of the invention and suitable for injection.

To improve the wettability of the microparticles of the invention, a wetting-agent can be included into the vehicle to improve the capacity of the microparticles of the invention to be suspended in an aqueous medium. However, by increasing their wettability, the microparticles then have the drawback that they sediment.

The vehicle may contain excipients, e.g. a viscosity-increasing agent and/or a wetting-agent. A vehicle for suspension may comprise a viscosity increasing agent and/or wetting agent as mentioned above and additionally water.
A suitable anti-agglomerating agent includes mannitol. Mannitol may also serve as a suitable isotonizing agent. If desired the pharmaceutical composition in the dry state may comprise an anti-agglomerating agent such as mannitol. Preferably, this is present in about2 to 10%, e.g. about 2-5%, e.g. 4% weight/weight of the microparticles in the dry state of the pharmaceutical composition.

The total concentration of the isotonizing agent corresponds to the concentration of the isotonizing agent when the microspheres are in suspension in the vehicle. The total concentration of isotonizing agent may be of from 1 to 50 mg/ml, e.g. 4 to 10 mg/ml, e.g. 5 to 8 mg/ml, e.g. 6 mg/ml. An anti-agglomerating agent can used in the pharmaceutical composition, i.e. the anti-agglomerating agent may be present in the dry state with the micropheres or may be present on the surface of the microspheres. If the agglomerating agent may be used as an isotonizing agent then the concentration of said isotonizing agent in the vehicle is calculated to so that the total concentration of isotonizing/anti-agglomerating agent in the vehicle with the microparticles in suspension in said vehicle may be in the range of from 1 to 50 mg/ml, e.g. 4 to 10 mg/ml, e.g. 5 to 8 mg/ml, e.g. 6 mg/ml.

A suitable viscosity increasing agent includes carboxymethyl cellulose sodium, herein after referred as NaCMC. NaCMC has a low viscosity. Embodiments may be as described above. Typically, NaCMC has a high molecular weight. The viscosity may be from about 1 to 30 mPa s, e.g. from 10 to about 15 mPa s when measured as a 1% (w/v) aqueous solution at 25°C in a Brookfield LVT viscometer with a spindle 1 at 60 rpm. Conveniently, the degree of substitution is of NaCMC from about 0.7 to about 1.45, e.g. from about 1.15 to about 1.45. Typically the sodium content of NaCMC is from about 5 to about 12%, e.g. about 10.5% to about 12%. NaCMC can be present in this is present in about 0.1-1%, e.g. 0.5% w/v of the vehicle composition. Said suitable viscosity-increasing agent can be present in a concentration of 1 to 30 mg/ml in the vehicle, e.g. 7 mg/ml, 10 mg/ml.

Preferably a wetting agent is present. Such wetting agents preferably include non-ionic surfactants.
a) Poloxamers also known as polyoxyethylene polyoxypropylene block copolymers Preferably, the poloxamers of the invention are solid.
   In one embodiment the molecular weight is from about 2000 to about 8000 daltons. The degree of polymerization of the ethylene moeity is typically 80 to about 110 units. The degree of polymerization of the propylene moiety is typically 20 to about 60 units. Examples of such compounds suitable for use in accordance with the present invention are those known and commercially available, e.g. under the trade name Pluronic F 68 available from BASF Germany. Pluronic F 68 can be present in the vehicle composition at a concentration of from 0.1 mg/ml to 5 mg/ml.
b) Polyoxyethylene-sorbitan-fatty acid esters e.g. mono- and trilauryl, palmityl, stearyl and oleyl esters e.g. of the type known and commercially available under the trade name TWEEN
   20 [polyoxyethylene(20)sorbitanmonolaurate],
   40 [polyoxyethylene(20)sorbitanmonopalmitate],
   60 [polyoxyethylene(20)sorbitanmonostearate],
   80 [polyoxyethylene(20)sorbitanmonooleate],
   65 [polyoxyethylene(20)sorbitantristearate],
   85 [polyoxyethylene(20)sorbitantrioleate],
   21 [polyoxyethylene(4)sorbitanmonolaurateJ,
   61 [polyoxyethylene(4)sorbitanmonostearatel, and
   81 [polyoxyethylene(5)sorbitanmonooleate].
Especially preferred products of this class for use in the pharmaceutical compositions of the invention are the above products TWEEN 2Q, TWEEN 40 and TWEEN 80. Such wetting agents are preferably present in about 0.01 to about 1% w/v, e.g. 0.1% w/v of the pharmaceutical composition. Said wetting agents may be present in about 0.01 to 5 mg/ml of the vehicle, e.g. 2 mg/ml.

The pharmaceutical composition may be stored under aseptic conditions, e.g. in a vial. All steps are conveniently effected under sterile conditions using sterile material, e.g. produced using sterile filters.
Microparticles of the present invention may be stored in the form of a powder. If desired a dry pharmaceutical composition and an aqueous medium vehicle for reconstitution may be housed separately in a double chamber syringe. For administration as injection the microparticles are suspended in a suitable vehicle for suspension, e.g. before administration to the patient.

The amount of liquid vehicle for suspension is preferably about 0.5 to 5 ml, e.g. 1 to 5 ml, e.g. 1 ml per dose, 2 ml per dose. The liquid may be mixed with the dry pharmaceutical composition just prior to administration.

The vehicle according to the present invention comprises a wetting agent in a concentration of 0.1 mg/ml to 5 mg/ml and/or a viscosity-increasing agent present in a concentration of 1 to 30 mg/ml and

In the further aspect the invention provides a pharmaceutical composition comprising octreotide acetate microparticles of linear poly (lactide-co-glycolide) polymer admixed or in association with a non-ionic surfactant (herein these compositions are also referred to as compositions of the invention).

Excipients disclosed in the literature, as for instance in the components of the compositions of the invention may be described in Fiedler, H.P. "Lexikon der Hilfsstoffe für Pharmazie, Kosmetik und angrenzende Gebiete", Editio Cantor Verlag Aulendorf, Aulendorf, 4th revised and expanded edition (1996) Germany and "Handbook of Pharmaceutical Excipients", Edited by A.H. Kibbe, American Pharmaceutical Association, Third Edition (2000), as well as manufacturer's brochures, may be used in the pharmaceutical compositions according to the invention.

The pharmaceutical compositions of the invention may be administered by intragluteal, intramuscular or subcutaneous injection. The pharmaceutical compositions of the invention administered by injection provide an effective treatment of diseases over an extended period, e.g. over 2 weeks to 6 months, e.g. over 2 weeks to 8 weeks, e.g. over 2 to 6 weeks. The microparticles allow a controlled release of octreotide by diffusion and therefore steady-state levels of the drug are obtained over the extended period.
The microparticles of the invention may be used for the same indications as known octreotide acetate microparticles.

The exact dose of octreotide will depend on a number of factors, including the condition to be treated, the severity of the condition to be treated, the weight of the subject and the duration of therapy.

The exact dose of microparticles of the invention used will depend on a number of factors, including the rate of release of octreotide and the desired duration of treatment.

The amounts may be determined using standard animal and clinical tests, e.g. bioavailability tests using rabbits, using SANDOSTATIN LAR as a standard. Octreotide levels may be determined using conventional methods, e.g. gas chromatography or high performance liquid chromatography. Typically for SANDOSTATIN LAR in humans an initial drug burst is seen, declining to a nadir in the next few days, followed by a plateau phase for 2 to 3 weeks post injection. For example at the 20 mg octreotide dose maximum serum concentrations of about 800 ng/l may be reached from day 21 and last for 4 weeks.

If desired conventional *in vitro* tests may be used. In one such test SANDOSTATIN LAR in acetate buffer pH 4 at 0.1 mM a continuous release profile is observed. Preferably pure water is used. Release characteristics of octreotide may be any of the following:
Not more than 1.5% of octreotide dose in one hour, e.g. at least 0.2%
Not more than 4% of octreotide dose in 4 hours; or
Not more than 7% of octreotide dose in 24 hours.

It is preferred to use single administration of the microparticles of the invention over 30 days. octreotide.

Conveniently the loading of octreotide in the microparticles of the invention is from about 1 to about 50 %, e.g. 1 to about 50%, e.g. 1 to 7%, e.g. from about 3 to about 7%, typically 4 to 6 %, e.g. 5%.

The pharmaceutical compositions of the invention are useful for the treatment of a disease treatable by octreotide, e.g. acromegaly.

The present disclosure also relates to:
a) Use of the octreotide for the manufacture of microparticles or pharmaceutical compositions of the invention to be administered to a patient for the treatment of a disease treatable by octreotide, e.g. acromegaly.
b) A method of administering of the octreotide, e.g. for the treatment of acromegaly, said method comprising administering to a patient in need of octreotide therapy microparticles or a pharmaceutical composition of the invention.

Following is a description by way of example only of depot formulations of this invention.

### Example 1: Microparticles

Step a): Approximately 2.5 g of poly(DL-lactide-co-glycolide) [polymer] are dissolved in 25 g methylene chloride to prepare a 9 wt. % polymer solution. After the polymer is completely dissolved, 188 mg octreotide acetate [drug] in 3.7 g methanol are added and allowed to dissolve.

Step b): This polymer/drug solution is then poured into a 1-L vessel containing 400 g of 5.0 wt. % polyvinyl alcohol (PVA). The PVA is stirred at about 750 rpm by a 2.5 inch impeller (e.g. TEFLON driven by a Fisher Stedi-speed motor). The PVA is also saturated with 7 ml of methylene chloride prior to the addition of the polymer/drug solution. The resulting emulsion is allowed to stir for 7 min.

Step c): The vessel contents are transferred all at once to 12.0 litres of stirred deionized water. The microparticles are stirred in the deionized water for approximately 30 min and then were collected over 45-µm mesh size meter and 212-µm mesh size stainless steel mesh steel sievs arranged in series. The microparticles are rinsed with additional deionized water and allowed to air dry.

The microparticles of the invention obtained have the characteristics described above.

### Example 2

### Vehicle composition

The microparticles of example 1 are mixed with mannitol and aseptically filled into two chamber syringe (TCS) consisting of one compartment containing the microparticles and the mannitol and one compartment containing the vehicle for suspension of the microparticles.

### Vehicle composition:

| | mg/ml | mg/ml |
|---|---|---|
| Pluronic F68 | 2.0 | 2.0 |
| Sodium-carboxymethylcellulose | 10.0 | 10.0 |
| (Blanose 7LFD) | | |
| Mannitol | 6.0 | 12.0 |
| Water for injections | ad 2.0 ml | ad 2.0 ml |

The components of the vehicle are mixed together under inert atmosphere, e.g. nitrogen. The vehicle for a 10 mg dose of octreotide in the microparticles of the invention for a dry pharmaceutical composition. 2 ml of vehicle are provided.

### Example 3: Vehicle in ampoule and microparticles in vials:

The 480 mg microparticles corresponding to a 20 mg dose of octreotide acetate with 5 % loading of example 1 are suspended in 2.0 ml of a vehicle of composition A below in 6R vials. The suspension is homogenized by shaking for about 30 seconds. The reconstituted suspension may be injected without any issues using a 20 Gauge needle.

### Vehicle composition A:

| | mg |
|---|---|
| Pluronic F68 | 2.0 |
| Sodium-carboxymethylcellulose | 7.0 |
| (Blanose 7LFD) | |
| Mannitol | 45.0 |
| Water for injections | ad 1.0 ml |

### Example 4: Microparticles and vehicle in the DCS

240 mg of microparticles of example 1 are reconstituted in 1 ml of the vehicle composition B, homogenized with a propeller mixer at 400 rpm for 1 to 12 hours and aseptically filled in the double chambers syringe then freeze-dried.

Reconstitution of the microparticle lyophilisates with 1 ml pure water (WBU) resulted in fast and good wetting of the microparticles that may be injected without any issues using a 20 Gauge needle.

### Vehicle composition B

| | mg |
|---|---|
| Pluronic F68 | 2.0 |
| Sodium-carboxymethylcellulose | 17.5 |
| (Blanose 7LFD) | |
| Mannitol | 36.0 |
| Water for injections | ad 1.0 ml |

## Claims

1. A process for the production of octreotide acetate microparticles comprising the steps of:
a) mixing octreotide acetate in methanol with methylene chloride containing a dissolved linear poly (lactide-co-glycolide) to form a solution; and
b) emulsifying said solution with the extraction medium, wherein said extraction medium is water or an aqueous buffered solution with a stabilizer;
c) immediately after the formation of emulsion, adding all at once said emulsion to an effective amount of an extraction medium to extract methylene to form said microparticles, wherein said extraction medium is water or an aqueous phase;
and
d) collecting and drying the microparticles, e.g. freeze-drying or drying under vacuum.

2. A process for the production of octreotide acetate microparticles comprising the steps of:
a) mixing octreotide acetate in methanol with methylene chloride containing a dissolved linear poly (lactide-co-glycolide) to form a solution; and
b) mixing said solution with high shear stress with a suitable quantity of process medium in the ratio of 1 volume of said solution of step a) with 10 to up to 50 volumes of process medium, wherein said process medium is an aqueous phase;
c) hardening the microparticles by solvent evaporation under stirring; and
d) washing, collecting and drying the microparticles.

3. The process of claim 1 or 2wherein the process or extraction medium comprises a stabilizing agent.

4. The process of claim 3, wherein the stabilizing agent is polyvinyl alcohol, polyvinyl pyrrolidone, or carboxymethyl cellulose sodium (NaCMC).

5. The process of claim 3, wherein the stabilizing agent is polyvinyl alcohol.

6. The process of any one of the claims 3 to 5, wherein the stabilizing agent in the process or the extraction medium is from 0.01% to about 20% w/v.

7. The process claim 6, wherein the stabilizing agent in the process or the extraction medium is from 0.025 to about 1%w/v.

8. The process of any one of the claims 2 to 7, wherein the process medium is saturated by methylene chloride.

## Patentansprüche

1. Verfahren zur Herstellung von Octreotidacetatmikropartikeln, umfassend folgende Schritte:
a) das Mischen von Octreotidacetat in Methanol mit Methylenchlorid enthaltend ein gelöstes lineares Poly(lactid-co-glycolid), um eine Lösung zu bilden; und
b) das Emulgieren der genannten Lösung mit dem Extraktionsmittel, wobei das genannte Extraktionsmittel Wasser oder eine wässrige gepufferte Lösung mit einem Stabilisator ist;
c) unmittelbar nach der Bildung der Emulsion, das Hinzufügen auf einmal der genannten Emulsion zu einer wirksamen Menge von einem Extraktionsmittel, um Methylen zu extrahieren, um die genannten Mikropartikel zu bilden, wobei das genannte Extraktionsmittel Wasser oder eine wässrige Phase ist;
und
d) das Einsammeln und das Trocknen der Mikropartikeln, z.B. das Gefriertrocknen oder das Vakuumtrocknen.

2. Verfahren zur Herstellung von Octreotidacetatmikropartikeln, umfassend folgende Schritte:
a) das Mischen von Octreotidacetat in Methanol mit Methylenchlorid umfassend ein gelöstes lineares Poly(lactid-co-glycolid), um eine Lösung zu bilden; und
b) das Mischen der genannten Lösung mit hoher Scherspannung mit einer geeigneten Menge von Prozessmedium im Verhältnis von 1 Volumen der genannten Lösung aus Schritt a) zu 10 bis 50 Volumen des Prozessmediums, wobei das genannte Prozessmedium eine wässrige Phase ist;
c) das Härten der Mikropartikeln durch Lösungsmittelverdampfung unter Rühren; und
d) das Waschen, das Einsammeln und das Trocknen der Mikropartikeln.

3. Verfahren nach Anspruch 1 oder 2, wobei das Verfahren oder das Extraktionsmittel ein Stabilisierungsmittel umfasst.

4. Verfahren nach Anspruch 3, wobei das Stabilisierungsmittel Polyvinylalkohol, Polyvinylpyrrolidon, oder Natriumcarboxymethylcellulose (NaCMC) ist.

5. Verfahren nach Anspruch 3, wobei das Stabilisierungsmittel Polyvinylalkohol ist.

6. Verfahren nach einem der Ansprüche 3 bis 5, wobei das Stabilisierungsmittel im Verfahren oder im Extraktionsmittel von 0,01% bis ca. 20% (w/v) beträgt.

7. Verfahren nach Anspruch 6, wobei das Stabilisierungsmittel im Verfahren oder im Extraktionsmittel von 0,025 bis ca. 1% (w/v) beträgt.

8. Verfahren nach einem der Ansprüche 2 bis 7, wobei das Prozessmedium mit Methylenchlorid gesättigt ist.

## Revendications

1. Procédé de production de microparticules d'acétate d'octréotide comprenant les étapes de:
a) mélanger de l'acétate d'octréotide dans du méthanol avec du chlorure de méthylène contenant un poly(lactide-co-glycolide) linéaire dissout pour former une solution; et
b) émulsionner ladite solution avec le moyen d'extraction, dans lequel ledit moyen d'extraction est de l'eau ou une solution tampon aqueuse avec un stabilisateur;
c) immédiatement après la formation de l'émulsion, ajouter en un fois ladite émulsion à une quantité effective d'un moyen d'extraction pour extraire du méthylène pour former lesdites microparticules, dans lequel ledit moyen d'extraction est de l'eau ou une phase aqueuse; et
d) recueillir et sécher les microparticules, par exemple, lyophiliser ou sécher sous vide.

2. Procédé de production de microparticules d'acétate d'octréotide comprenant les étapes de:
a) mélanger de l'acétate d'octréotide dans du méthanol avec du chlorure de méthylène contenant un poly(lactide-co-glycolide) linéaire dissout pour former une solution; et
b) mélanger ladite solution avec un cisaillement élevé avec une quantité appropriée de moyen de procédé dans la proportion d'1 volume de ladite solution de l'étape a) avec 10 jusqu'à 50 volumes du moyen de procédé, dans lequel ledit moyen de procédé est une phase aqueuse;
c) durcir les microparticules par l'évaporation du solvant sous agitation; et
d) laver, recueillir et sécher les microparticules.

3. Procédé selon les revendications 1 ou 2 dans lequel le moyen de procédé ou d'extraction comprend un agent de stabilisation.

4. Procédé selon la revendication 3, dans lequel l'agent de stabilisation est du poly(alcool vinylique), de la polyvinylpyrrolidone, ou de la carboxyméthylcellulose sodique (NaCMC).

5. Procédé selon la revendication 3, dans lequel l'agent de stabilisation est du poly(alcool vinylique).

6. Procédé selon l'une quelconque des revendications 3 à 5, dans lequel l'agent de stabilisation dans le moyen de procédé ou d'extraction est de 0,01% à environ 20% p/v.

7. Procédé selon la revendication 6, dans lequel l'agent de stabilisation dans le moyen de procédé ou d'extraction est de 0,025 à environ 1% p/v.

8. Procédé selon l'une quelconque des revendications 2 à 7, dans lequel le moyen de procédé est saturé par du chlorure de méthylène.
